# EUROPEAN PATENT SPECIFICATION

(11) **EP 2 732 771 B1**
(45) Date of publication and mention of the grant of the patent: **09.09.2015**
(21) Application number: 13191461.6
(22) Date of filing: 04.11.2013
(51) Int. Cl.: A61B 17/00

(54) **Degradable balloon device for closure of openings in a tissue wall**
Abbaubare Ballonvorrichtung zum Verschließen von Öffnungen in einer Gewebewand
Dispositif à ballonnet dégradable pour fermeture d'ouvertures dans une paroi tissulaire

(30) Priority: 19.11.2012 US 201261727958 P
(43) Date of publication of application: 21.05.2014
(73) Proprietor: Cook Medical Technologies LLC, Bloomington, IN 47404 (US)
(72) Inventor: Marchi, Benjamin, West Lafayette, IN Indiana 47906 (US); Kratzberg, Jarin, Lafayette, IN Indiana 47909 (US)
(74) Representative: Williams Powell

(56) References cited:
- WO-A1-2010/085449
- US-A- 5 630 833
- US-A1- 2008 097 509
- US-A1- 2009 099 589

## Description

### BACKGROUND

The present invention relates to closure of openings in tissue walls percutaneously, and more specifically to devices therefor with one or more balloons.

Percutaneous access provides minimally invasive access to subcutaneous tissues walls, such as for example vascular access without excessive cut downs or other invasive techniques. Access is acquired by a needle puncture through the skin into the desired vessel. A guide wire is then inserted into the vessel through the access site. The initial puncture is expanded through progressive dilation over the guide wire; the access site is dilated such that the desired endovascular device can be inserted into the vessel. Once the procedure is complete, the delivery system of the endovascular device is removed through the access site. However, once the delivery system is withdrawn from the vessel a lesion remains. The device described herein provides temporary hemostatis around the access site (specifically the lesion in the vessel wall), and promotes healing of the native vessel with small intestine submucosa (SIS).

Current percutaneous closure devices are typically limited to use in peripheral access cases (access site size typically less than or equal to 10 Fr), and some require permanent implantation; direct pressure on the access site is also used to provide hemostasis. Thus, there is a need for improvement in this field.

US5630833 A (KATSAROS GEORGES [BE] ET AL, 1997) discloses a device for sealing hemostatic incisions comprising a first inflatable and bioabsorbable balloon, a second inflatable and bioabsorbable balloon, longitudinally and proximally spaced apart from the first balloon and a collagen member located between the balloons.

### SUMMARY

The present invention seeks to provide an improved device for closure of an opening in a patient's subcutaneous tissue wall.

According to an aspect of the present invention, there is provided a device for closure of an opening in a medical patient's subcutaneous tissue wall as specified in claim 1.

Another aspect of the disclosure provides a method including the step of inserting into a patient such a device.

According to another aspect of the present disclosure there is provided a method for closure of an opening in a medical patient's subcutaneous tissue wall, including the steps of: inserting a catheter having: (a) a first inflatable, bio-degradable balloon and a sheet of extracellular matrix material loaded on and around said catheter and located proximal of said first balloon; and, (b) a second inflatable, bio-degradable balloon of said catheter located proximal of said first balloon and a second sheet of extracellular matrix material loaded on and around said catheter and located distal of said second balloon, with said first balloon and said first sheet of extracellular matrix material through the opening in the tissue wall; such that upon inflation ofsaid first and second balloons, the first sheet of extracellular matrix material is held against the tissue wall by said first balloon and the second sheet of extracellualr matrix material is held against the tissue wall by said second balloon, said sheets of extracellular matrix material being pressed together by said first and second balloons.

The teachings herein extend to any device or apparatus comprising means suitable for carrying out the taught method steps.

As merely a summary, the preferred embodiment includes a device for closure of an opening in a medical patient's subcutaneous tissue wall. The device includes a catheter having two inflatable, bio-degradable balloons. It also includes two sheets of extracellular matrix material loaded on and around said catheter, wherein upon inflation of the balloons, the sheets of extracellular matrix material are held against the tissue wall by the balloons.

The present disclosure may also include surgical insertion methods of the various devices shown and described.

Further forms, objects, aspects, benefits, advantages and embodiments of the present invention will become apparent from a detailed description and drawings provided herewith.

### BRIEF DESCRIPTION OF THE DRAWINGS

Embodiments of the present invention are described below, by way of example only, with reference to the accompanying drawings, in which:
FIG. 1 is a perspective view of one embodiment of medical device;
FIGS. 2A-2F are side cut away views of the insertion of the device of FIG. 1;
FIG. 3 is partial cut away view of one example of a catheter of the device of FIG. 1;
FIG. 4 is a perspective view of one example of a sheet of extracellular matrix material shown in isolation;
FIG. 5 is a side cross sectional view of another example of two sheets (joined) of extracellular matrix material shown in isolation; and
FIG. 6 is partial cut away perspective detail of another example of the device of FIG. 1.

### DESCRIPTION OF THE SELECTED EMBODIMENTS

For the purpose of promoting an understanding of the principles taught herein, reference will now be made to the embodiments illustrated in the drawings and specific language will be used to describe the same. It will nevertheless be understood that no limitation of the scope of the invention is thereby intended. Any alterations and further modifications in the described embodiments, and any further applications of the principles of the invention as described herein are contemplated as would normally occur to one skilled in the art to which the invention relates. One embodiment of the invention is shown in great detail, although it will be apparent to those skilled in the relevant art that some features that are not relevant to the present invention may not be shown for the sake of clarity.

Percutaneous access provides minimally invasive vascular access without excessive cut downs or other invasive techniques. Access is acquired by a needle puncture through the skin into the desired vessel. A guide wire is then inserted into the vessel through the access site. The initial puncture is expanded through progressive dilation over the guide wire; the access site is dilated such that the desired endovascular device can be inserted into the vessel. Once the procedure is complete, the delivery system of the endovascular device is removed through the access site. However, once the delivery system is withdrawn from the vessel a lesion remains. The device described herein provides temporary hemostatis around the access site (specifically the lesion in the vessel wall), and promotes healing of the native vessel with SIS.

Current percutaneous closure devices are limited to use in peripheral access cases (access site size typically less than or equal to 10 Fr), and some require permanent implantation; direct pressure on the access site is also used to provide hemostasis. The proposed device allows for closure from large French size delivery systems typically used in endovascular aneurysmal and abdominal arterial repairs. This device also performs the closure with no long term implantation. The device design will target closure of 6-24 Fr percutaneous access.

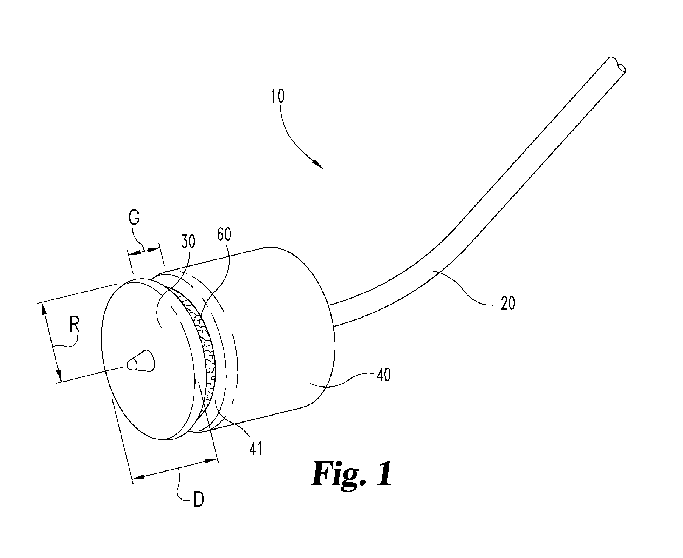

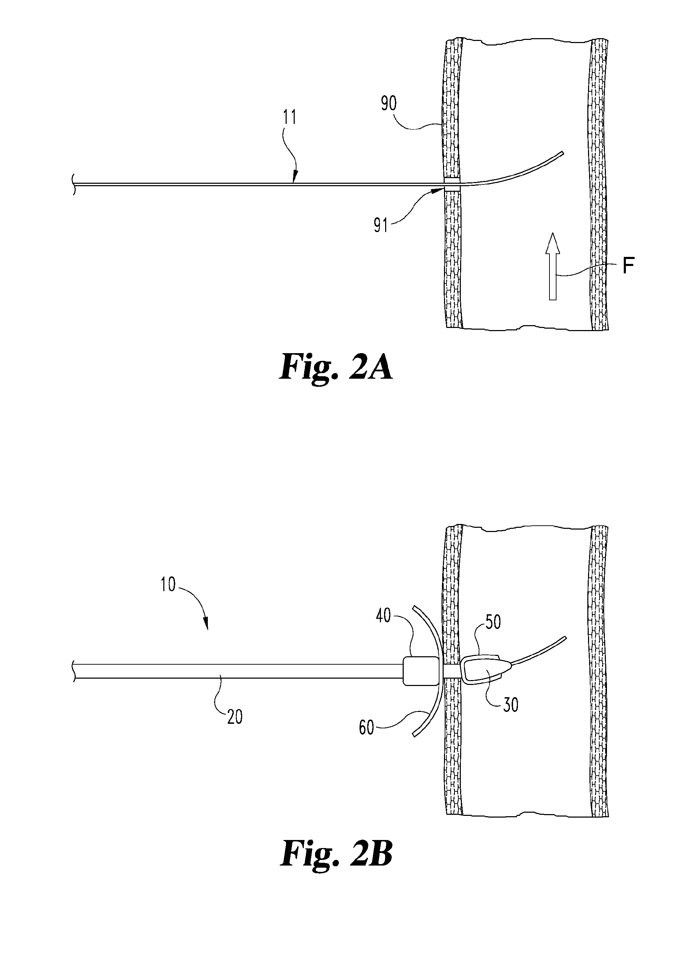

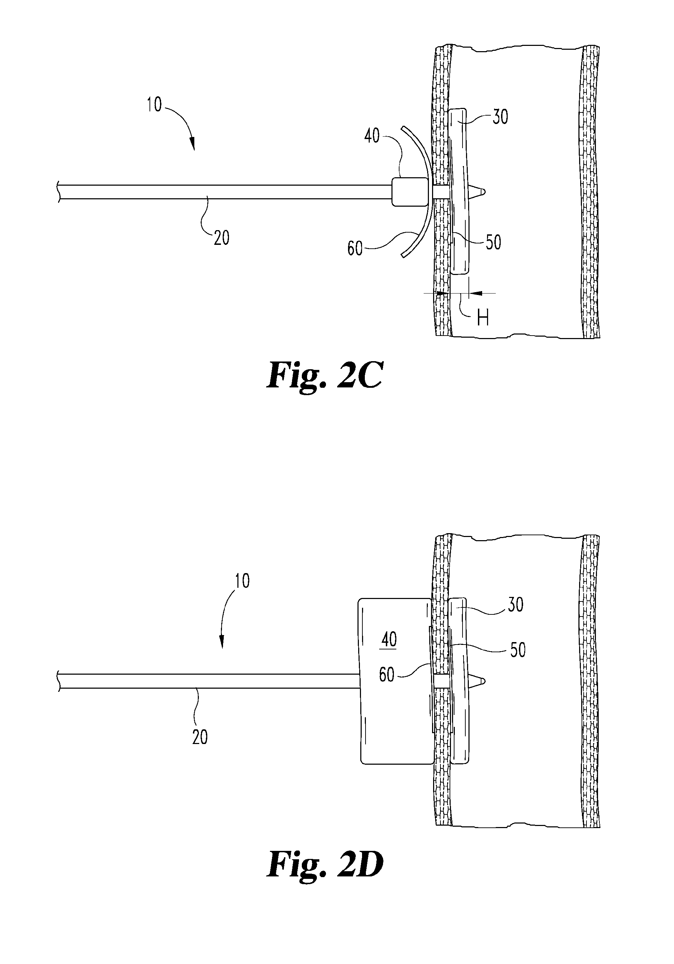

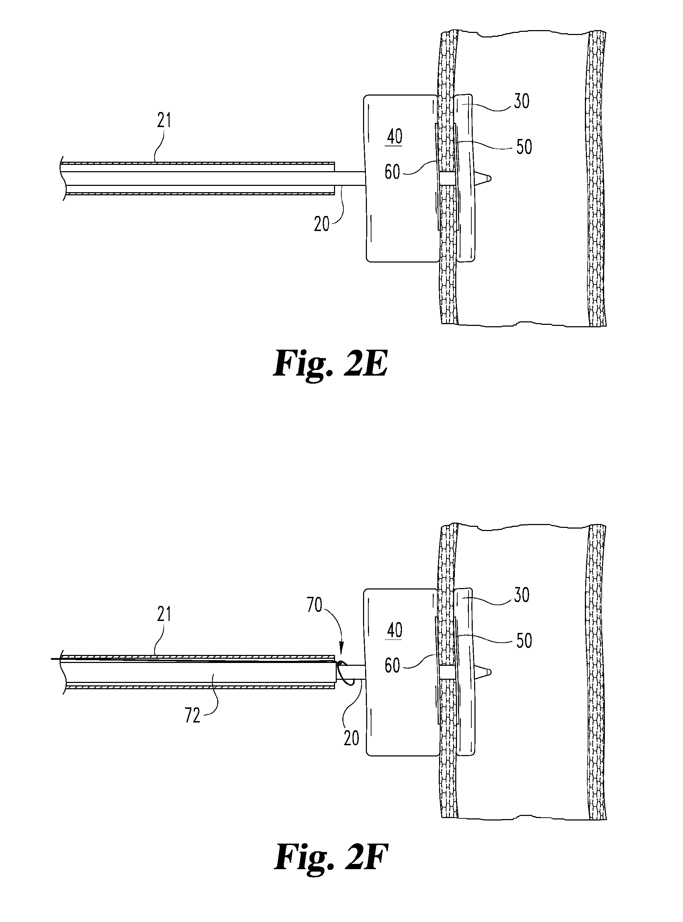

The proposed design includes a balloon catheter, knot pusher, and a decoupler. These components are housed typically in a 12 Fr sheath, although other sizes may be used. There are two balloons on the catheter. The distal balloon is situated near the tip of the catheter. This balloon is disk like in shape (FIGS. 1 and 2); the balloon has a diameter larger than the lesion (approximately 3 mm greater than the outer diameter of the original device delivery system), and a height such that the protrusion into the vessel does not negatively affect the blood flow, namely occupy less than 40% of the cross-sectional area of the vessel's lumen. This geometry promotes good sealing of the lesion, as well as protection against migration. The proximal balloon also has a cylindrical shape, and has the same diameter as the distal balloon. The height of the proximal balloon is 2-3 times greater than the distal balloon. The balloons are positioned on the catheter such that there is a 1-2 mm gap between the expanded balloon profiles. Hemostasis is achieved when the proximal balloon is expanded. The size of the gap between the balloons, along with the balloon geometry, promotes sealing against both sides of the vessel. An SIS disk is fixed to both the proximal surface of the distal balloon and the distal surface of the proximal balloon (FIGS. 1 and 2). When the balloons are expanded the SIS will be in contact with both sides of the vessel wall to promote healing of the native vessel. Both the balloons and the catheter will be made of biodegradable/bioabsorbable polymer.

A pre-tied sliding and self-locking knot will be looped over the catheter proximal to the proximal balloon. The post of the knot will be threaded through the lumen of a stiff cannula pusher. The pusher will be used to tighten the knot onto the catheter. The knot will be tied with an absorbable suture. The knot pusher will extend past the sheath, and it will be manipulated by the physician to achieve proper knot positioning. When a decoupler is employed, it will cut the catheter proximal to the knot. A possible decoupler may be formed of coiled wires which, when current is applied, fuse and sever the catheter stem.

The proposed device allows for closure from large French size delivery systems typically used in endovascular aneurysmal and abdominal arterial repairs. This device also performs the closure with no long term implantation. The device design will target closure of typically 6-24 Fr, and preferably 12-24 Fr, percutaneous access.

The term "bio-degradable" means able to be broken down and absorbed in, resorbed and/or passed from the human body.

The term "central hole" means an opening (round, slit(s), rectangular or any other shape) in a sheet or other member that is at least partially within about the central two-thirds of the shape.

The term "closing a lumen" means reducing its cross-section at one or more points sufficient to substantially restrict fluid flow through the lumen. This may be done with one or more knot, noose, check valve, plug, flap and/or other structure.

The term "cutting off" means fully or substantially severing one part so it may be separated from another.

The term "depth" means the average distance of an element taken in a direction proximal to distal.

The term "distal" means, relative to a reference, away from the doctor or other operator of the device; normally the opposite of "proximal".

The term "extracellular matrix material" means material harvested from a (previously) living animal which is an extracellular matrix, typically including collagen. This includes without limitation material harvested from the walls of animal intestine, bladder, liver, and stomach.

The term "loaded" means placed into position prior to insertion into a patient.

The term "proximal" means, relative to a reference, towards the doctor or other operator of the device; normally the opposite of "distal".

The term "radius" means the average distance of an element taken in a direction from the center to the perimeter.

The term "sheet" means a generally flat member which is thinner in depth than in the other dimensions and is at least somewhat flexible. It may be single layer, multi-layer, formed, cut, pressed, molded, porous, non-porous, and/or otherwise. A sheet may be round (such as a round disk), rectangular, square, elliptical, hexagonal, octagonal, oblong, irregular or any other shape.

The term "small intestine submucosa" means material (sheet, solid shape, powder, liquid or otherwise) which is entirely or partially made up of portions of the submucosal layer of an animal's small intestines.

The term "subcutaneous tissue wall" means any tissue wall of a patient beneath the skin, including without limitation blood vessel walls.

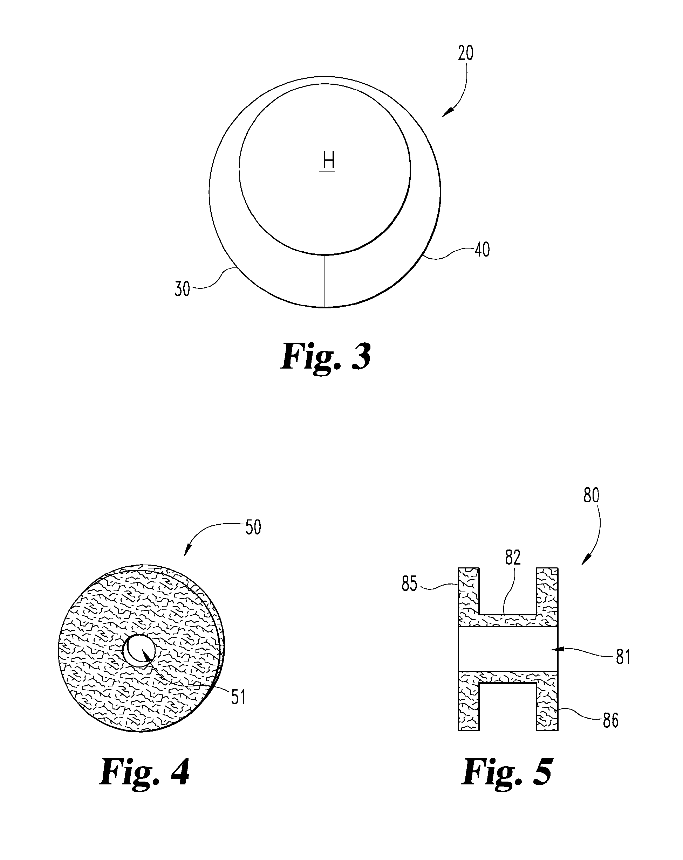

The proposed device 10 may comprise a balloon catheter 20, an optional knot pusher 72 (see FIG. 2F), and an optional decoupler. The balloons and sheets may be mounted and dismountable from the catheter. These components are typically housed in a sheath 21, at least during part of installation, such as for example a 12 Fr sheath. The catheter may have lumen L advance-able over a wire guide 11. There are normally two balloons 30 and 40 on the catheter. The distal balloon 30 is situated at or near the tip of the catheter. This balloon, when inflated, may be disk like in shape (*see* FIGS. 1 and 2C); the balloon has a diameter D (twice radius R)(*see* FIG. 1) larger than the lesion or opening 91 in tissue wall 90 (FIG. 2A) (the diameter typically approximately 3 mm greater or more than the outer diameter of the original device delivery system), and a depth, (also referred to herein as height H) (*see* FIGS. 2C and 2E) such that the protrusion into the vessel does not negatively affect any fluid flow, such as for example blood flow F (*see* FIG. 2A). This geometry promotes good sealing of the lesion, as well as protection against migration. The proximal balloon 40, when inflated, also may have a cylindrical (or other) shape, and typically has about the same diameter as the distal balloon 30. The depth of the proximal balloon may be smaller, the same as or greater, for example 2-3 times greater, than the distal balloon. Normally it is greater as illustrated. The balloons are positioned on the catheter such that there is a 1-2 mm gap G (*see* FIGS. 1 and 2D) between the expanded balloon profiles. Hemostasis or other closure may be achieved when the proximal balloon is expanded. The size of the gap between the balloons, along with the balloon geometry, promotes sealing against both sides of the vessel. An extracellular matrix (extracellular matrix) material sheet 50, 60, in the shape of a disk or otherwise, is loaded on, and optionally fixed to, one or both of the proximal surface of the distal balloon and the distal surface 41 of the proximal balloon (FIGS. 1 and 2). The sheet may be any shape, with a round disk example shown in FIG. 4 with a central opening 51 to facilitate loading onto the catheter. When the balloons are expanded the extracellular matrix will be in contact with both sides of the vessel wall 90 to promote healing of the native vessel. Both the balloons and the catheter will be made of bio-degradable material, including bioabsorbable material, such as a bio-degradable polymer.

An optional type of alternative of a sheet of FIG. 4 is shown in FIG. 5, this being a side cross-sectional view. There, implant 80 comprises at least two extracellular matrix material sheets 85, 86 joined by connection 82. Connection 82 may be extracellular matrix material or other bio-degradable material. Lumen 81 may receive the catheter through it so as to provide a balloon on the distal side of implant 80.

A pre-tied sliding and self-locking knot 70 may be looped over the catheter proximal to the proximal balloon. The post of the knot may be threaded through the lumen of a stiff cannula pusher 72. The pusher will be used to tighten the knot onto the catheter. The knot may be tied with an absorbable suture. The knot pusher can extend past the sheath and be manipulated by the physician to achieve proper knot positioning. It may be tightened for closing a lumen, such as to close one or more lumens in the catheter maintaining balloons 30 and/or 40 inflated.

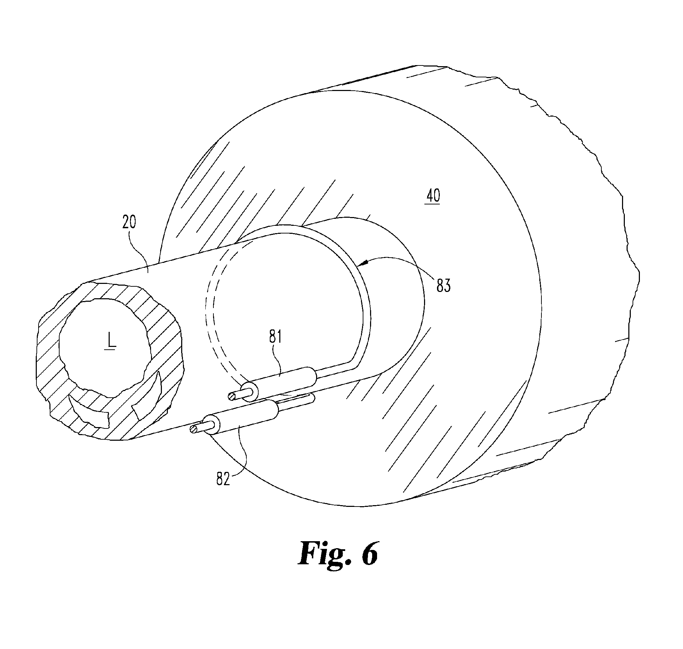

A decoupler may be used to cut the catheter proximal to the knot. A possible decoupler consists of coiled wire(s) 81, 82, 83 (*see* FIG. 6) which, when electric current is applied, provide for cutting the catheter stem, preferably proximal to the elements that close the lumens.

The following is a summary of one example that can be used for the delivery procedure for the devices disclosed herein:
1. Remove delivery system of the last endovascular device used in the repair, while maintaining vessel access with the guide wire.
2. Prep the proposed closure device's balloons, and flush the catheter with saline.
3. Advance the device until into the desired vessel.
4. Expand the distal balloon while inside the vessel. Then pull back on the sheath to verify that the inflated balloon can provide adequate closure of the lesion.
5. While maintaining tension against the inner side of the vessel wall, inflate the proximal balloon. There should now be successful closure of the lesion or opening. Retract the guide wire.
6. Advance the pusher to tighten the knot.
7. Once the knot is tight, engage the decoupler to cut the catheter and extra suture.
8. Remove the remains of the severed delivery system from the access site.

While the invention has been illustrated and described in detail in the drawings and foregoing description, the same is to be considered as illustrative and not restrictive in character, it being understood that only the preferred embodiment has been shown and described and that all changes, equivalents, and modifications that come within the scope of the claims are desired to be protected.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

## Claims

1. A device for closure of an opening in a medical patient's subcutaneous tissue wall, including:
a catheter (20) having a first inflatable, bio-degradable balloon (30), a sheet of extracellular matrix material (50) loaded on and around said catheter and located proximal of said first balloon;
a second inflatable, bio-degradable balloon (40) located proximal of said first balloon (30); a second sheet of extracellular matrix material (60) loaded on and around said catheter (20) and located distal of said second balloon;
said device being configured such that tissue wall is locatable between said first (50) and second (60) sheets of extracellular matrix material and such that upon inflation of said first (30) and second (40) balloons, the first sheet of extracellular matrix material is held against the tissue wall by said first balloon and the second sheet of extracellular matrix material is held against the tissue wall by said second balloon, said sheets of extracellular matrix material being pressed together by said first and second balloons.

2. A device according to claim 1 including:
means for closing a lumen of said catheter (20) after one or more of the balloons (30, 40) of said catheter are inflated.

3. A device according to claim 2, wherein said means for closing includes a cord formed in a noose (70) to cinch said catheter.

4. A device according to any preceding claim, including means for cutting off said catheter (20) subcutaneously and distally of each of the catheter's balloons.

5. A device according to claim 4, wherein said means for cutting includes a wire (83) in said catheter (20) to conduct electrical current to generate cutting heat around a circumference of said catheter to weaken or sever the catheter subcutaneously.

6. A device according to any preceding claim, wherein said or at least one sheet of said extracellular matrix material (50, 60) is generally round in shape with a central hole (51) closely conforming to the outer diameter of said catheter (20).

7. A device according to any preceding claim, wherein said or at least one sheet of extracellular matrix material (50, 60) comprises small intestine submucosa.

8. A device according to any preceding claim, wherein at least a portion of said catheter (20) adjacent said one or more balloons (30, 40) is bio-degradable.

9. A device according to any preceding claim, wherein said first balloon (30) is generally disc shaped, having a depth less than a radius thereof.

## Patentansprüche

1. Vorrichtung zum Verschließen einer Öffnung in einer subkutanen Gewebewand eines medizinischen Patienten, umfassend:
einen Katheter (20) mit einem ersten aufblähbaren, biologisch abbaubaren Ballon (30), einem Blatt von extrazellulärem Matrixmaterial (50), das auf und um den Katheter geladen und proximal bezogen auf den ersten Ballon angeordnet ist;
einen zweiten aufblähbaren, biologisch abbaubaren Ballon (40), der proximal bezogen auf den ersten Ballon (30) angeordnet ist; ein zweites Blatt von extrazellulärem Matrixmaterial (60), das auf und um den Katheter (20) geladen und distal bezogen auf den zweiten Ballon angeordnet ist;
wobei die Vorrichtung so gestaltet ist, dass eine Gewebewand zwischen dem ersten (50) und dem zweiten (60) Blatt von extrazellulärem Matrixmaterial angeordnet werden kann, und so, dass bei Aufblähen des ersten (30) und des zweiten (40) Ballons das erste Blatt von extrazellulärem Matrixmaterial durch den ersten Ballon gegen die Gewebewand gehalten wird und das zweite Blatt von extrazellulärem Matrixmaterial durch den zweiten Ballon gegen die Gewebewand gehalten wird, wobei die Blätter von extrazellulärem Matrixmaterial durch den ersten und den zweiten Ballon aneinandergepresst werden.

2. Vorrichtung gemäß Anspruch 1, umfassend:
Mittel zum Verschließen eines Lumens des Katheters (20), nachdem einer oder mehrere der Ballons (30, 40) des Katheters aufgebläht worden sind.

3. Vorrichtung gemäß Anspruch 2, wobei das Mittel zum Verschließen eine zu einer Schlaufe (70) gebildete Leine zum Regeln des Katheters umfasst.

4. Vorrichtung gemäß einem der vorstehenden Ansprüche, umfassend Mittel zum subkutanen Abschneiden des Katheters (20) distal bezogen auf jeden der Ballons des Katheters.

5. Vorrichtung gemäß Anspruch 4, wobei das Mittel zum Schneiden einen Draht (83) in dem Katheter (20) umfasst, um elektrischen Strom zu leiten, um Schneidehitze um einen Umfang des Katheters zu erzeugen, um den Katheter subkutan zu schwächen oder zu trennen.

6. Vorrichtung gemäß einem der vorstehenden Ansprüche, wobei das oder wenigsten ein Blatt des extrazellulären Matrixmaterials (50, 60) allgemein rund geformt ist und ein zentrales Loch (51) aufweist, das dem Außendurchmesser des Katheters (20) eng entspricht.

7. Vorrichtung gemäß einem der vorstehenden Ansprüche, wobei das oder wenigsten ein Blatt von extrazellulärem Matrixmaterial (50, 60) Dünndarm-Submukosa umfasst.

8. Vorrichtung gemäß einem der vorstehenden Ansprüche, wobei wenigstens ein Teil des Katheters (20) benachbart zu dem einen oder den mehreren Ballons (30, 40) biologisch abbaubar ist.

9. Vorrichtung gemäß einem der vorstehenden Ansprüche, wobei der erste Ballon (30) allgemein scheibenförmig ist und eine Tiefe aufweist, die kleiner als sein Radius ist.

## Revendications

1. Dispositif pour fermer une ouverture dans une paroi tissulaire sous-cutanée d'un patient médical, comprenant:
un cathéter (20) comprenant un premier ballonnet biodégradable gonflable (30), une feuille de matière de matrice extracellulaire (50) chargée sur et autour dudit cathéter et située à proximité dudit premier ballonnet;
un deuxième ballonnet biodégradable gonflable (40) situé à proximité dudit premier ballonnet (30); une deuxième feuille de matière de matrice extracellulaire (60) chargée sur et autour dudit cathéter (20) et située à distance dudit deuxième ballonnet;
ledit dispositif étant configuré de telle sorte que la paroi tissulaire puisse être située entre lesdites première (50) et deuxième (60) feuilles de matière de matrice extracellulaire et de telle sorte que, lors du gonflage desdits premier (30) et deuxième (40) ballonnets, la première feuille de matière de matrice extracellulaire soit maintenue contre la paroi tissulaire par ledit premier ballonnet, et que la deuxième feuille de matière de matrice extracellulaire soit maintenue contre la paroi tissulaire par ledit deuxième ballonnet, lesdites feuilles de matière de matrice extracellulaire étant pressées ensemble par lesdits premier et deuxième ballonnets.

2. Dispositif selon la revendication 1, comprenant des moyens pour fermer une lumière dudit cathéter (20) après qu'un ou plusieurs des ballonnets (30, 40) dudit cathéter ai (en) t été gonflé (s).

3. Dispositif selon la revendication 2, dans lequel lesdits moyens de fermeture comprennent un cordon en forme de noeud (70) pour serrer ledit cathéter.

4. Dispositif selon l'une quelconque des revendications précédentes, comprenant des moyens pour sectionner ledit cathéter (20) de façon sous-cutanée et à distance de chacun des ballonnets du cathéter.

5. Dispositif selon la revendication 4, dans lequel lesdits moyens de coupe comprennent un fil (83) dans ledit cathéter (20) pour conduire un courant électrique afin de générer une chaleur de coupe autour d'une circonférence dudit cathéter dans le but d'affaiblir ou de trancher le cathéter de façon sous-cutanée.

6. Dispositif selon l'une quelconque des revendications précédentes, dans lequel ladite au moins une feuille de ladite matière de matrice extracellulaire (50, 60) est de forme essentiellement ronde avec un trou central (51) qui épouse étroitement le diamètre extérieur dudit cathéter (20).

7. Dispositif selon l'une quelconque des revendications précédentes, dans lequel ladite ou au moins une feuille de matière de matrice extracellulaire (50, 60) comprend la sous-muqueuse de l'intestin grêle.

8. Dispositif selon l'une quelconque des revendications précédentes, dans lequel au moins une partie dudit cathéter (20) située à proximité dudit/desdits un ou plusieurs ballonnet(s) (30, 40) est biodégradable.

9. Dispositif selon l'une quelconque des revendications précédentes, dans lequel ledit premier ballonnet (30) est essentiellement en forme de disque, avec une profondeur inférieure à un rayon de celui-ci.
